# EUROPÄISCHE PATENTSCHRIFT

(11) **EP 2 417 333 B1**
(45) Veröffentlichungstag und Bekanntmachung des Hinweises auf die Patenterteilung: **05.06.2019**
(21) Anmeldenummer: 10711055.3
(22) Anmeldetag: 25.03.2010
(51) Int. Cl.: F01M 1/08, F01M 11/02, F01M 11/10, F16N 29/00, G01M 15/04, G01N 33/22, G01N 33/28

(54) **ÜBERWACHUNGSVORRICHTUNG, SOWIE ÜBERWACHUNGSVERFAHREN ZUR ÜBERWACHUNG EINES VERSCHLEISSZUSTANDS EINER KOMPONENTE EINER HUBKOLBENBRENNKRAFTMASCHINE**
MONITORING DEVICE AND MONITORING METHOD FOR MONITORING A STATE OF WEAR OF A COMPONENT OF A RECIPROCATING INTERNAL COMBUSTION ENGINE
DISPOSITIF DE SURVEILLANCE ET PROCÉDÉ DE SURVEILLANCE SERVANT À SURVEILLER UN ÉTAT D'USURE D'UN ÉLÉMENT D'UN MOTEUR À COMBUSTION INTERNE À PISTON ALTERNATIF

(30) Priorität: 06.04.2009 EP 09157425
(43) Veröffentlichungstag der Anmeldung: 15.02.2012
(73) Patentinhaber: Wärtsilä Schweiz AG, 8401 Winterthur (CH)
(72) Erfinder: MICALI, Francesco, San Pietro Vernotico I.72027 Brindisi (IT); STARK, Matthias, CH-8400 Winterthur (CH); WEBER, Markus, CH-8604 Volketswil (CH)
(74) Vertreter: Intellectual Property Services GmbH
(86) Internationale Anmeldenummer: PCT/EP2010/053880
(87) Internationale Veröffentlichungsnummer: WO 2010/115716

(56) Entgegenhaltungen:
- EP-A1- 1 643 088
- WO-A1-03/048763
- JP-A- 5 163 919
- JP-A- 6 094 188
- JP-A- 62 243 912
- JP-A- 2005 299 459
- US-A- 4 306 525
- US-A1- 2001 013 247

## Beschreibung

Die Erfindung betrifft eine Hubkolbenbrennkraftmaschine mit einer Überwachungsvorrichtung, sowie einem Überwachungsverfahren zur Überwachung eines Verschleisszustands einer Komponente einer Hubkolbenbrennkraftmaschine gemäss dem Oberbegriff des unabhängigen Anspruchs der jeweiligen Kategorie.

Grossdieselmotoren werden häufig als Antriebsaggregate für Schiffe oder auch im stationären Betrieb, z.B. zum Antrieb grosser Generatoren zur Erzeugung elektrischer Energie eingesetzt. Dabei laufen die Motoren in der Regel über beträchtliche Zeiträume im Dauerbetrieb, was hohe Anforderungen an die Betriebssicherheit und die Verfügbarkeit stellt. Daher sind für den Betreiber insbesondere lange Wartungsintervalle, geringer Verschleiss und ein wirtschaftlicher Umgang mit Brenn- und Betriebsstoffen zentrale Kriterien für den Betrieb der Maschinen. Unter anderem ist das Kolbenlaufverhalten solcher grossbohrigen langsam laufenden Dieselmotoren ein bestimmender Faktor für die Länge der Wartungsintervalle, die Verfügbarkeit und über den Schmiermittelverbrauch auch unmittelbar für die Betriebskosten und damit für die Wirtschaftlichkeit. Damit kommt der komplexen Problematik der Schmierung von Grossdieselmotoren eine immer grössere Bedeutung zu.

Bei Grossdieselmotoren, jedoch nicht nur bei diesen, erfolgt die Kolbenschmierung durch Schmiereinrichtungen im sich hin und her bewegenden Kolben oder in der Zylinderwand, durch die Schmieröl auf die Lauffläche der Zylinderwand aufgebracht wird, um die Reibung zwischen Kolben und Lauffläche und damit die Abnützung der Lauffläche und der Kolbenringe zu minimieren. So liegt heute bei modernen Motoren, wie z.B. den RTA-Motoren von Wärtsilä, die Abnutzung der Lauffläche bei weniger als 0.05 mm bei einer Betriebsdauer von 1000 Stunden. Die Schmiermittelfördermenge liegt bei solchen Motoren bei ca. 1.3 g/kWh und weniger und soll nicht zuletzt aus Kostengründen möglichst noch weiter reduziert werden, wobei gleichzeitig der Verschleiss minimiert werden soll.

Als Schmiersysteme zur Schmierung der Laufflächen sind ganz verschiedene Lösungen bekannt, sowohl was die konkrete Ausführung der Schmiereinrichtungen selbst, als auch was die Verfahren zur Schmierung angeht. So sind Schmiereinrichtungen bekannt, bei denen das Schmieröl durch mehrere Schmiermittelöffnungen, die in Umfangsrichtung in der Zylinderwand untergebracht sind, auf den an der Schmiermittelöffnung vorbeilaufenden Kolben aufgebracht werden, wobei das Schmiermittel durch die Kolbenringe sowohl in Umfangsrichtung als auch in axialer Richtung verteilt wird. Das Schmiermittel wird bei dieser Methode nicht grossflächig auf die Lauffläche der Zylinderwand, sondern mehr oder weniger punktuell zwischen die Kolbenringe auf die Seitenflächen des Kolbens aufgebracht.

Ganz gleich nach welcher Methode das Schmieröl auf die Gegenlaufpartner aufgebracht wird, gibt es spezielle Probleme im Zusammenhang mit der Zylinderschmierung und vor allem mit dem Verschleiss der Komponenten von Kreuzkopf-Grossdieselmotoren, die bis heute ungelöst sind.

Dabei ist es bekannt zumindest grob den Verschleisszustand von Kolben, Kolbenringen, Gaswechselventilen wie etwa dem Auslassventil, der Lauffläche und anderen Komponenten der Brennkraftmaschine dadurch zu bestimmen, dass bestimmte Eigenschaften des in der Brennkraftmaschine verwendeten Schmieröls untersucht werden. Dazu wird Schmieröl, das sich zum Beispiel im Fall von längs gespülten Zweitakt-Grossdieselmotoren am Boden des Kolbenunterseitenraums niederschlägt, in einem Behälter gesammelt und das gesammelte Schmieröl z.B. auf Eisenpartikel untersucht. Wenn dann eine bestimmte Menge an Eisen im so gesammelten Schmieröl festgestellt wird, lässt dies gewisse grobe Rückschlüsse auf den Verschleisszustand der vorgenannten Zylinderkomponenten zu. Die Messung des Eisengehaltes erfolgt dabei in an sich bekannter Weise mit magnetischen Verfahren. JPS62243912 A und JPH0694188 A beschreiben auch Überwachungsverfahren für das Schmieröl einer Brennkraftmaschine.

Diese bekannten Verfahren haben aber eine Reihe von Nachteilen, die in der Praxis die Messergebnisse oft massiv verfälschen.

So ist das Schmieröl, das sich zum Beispiel am Boden des Kolbenunterseitenraums abgesetzt hat, mit vielen anderen Stoffen kontaminiert, die ursprünglich nicht aus dem Brennraum der Brennkraftmaschine stammen. So werden Verunreinigungen aus dem Turboladersystem in den Receiverraum transportiert, die das Schmieröl zusätzlich verunreinigen. Auch durch die Stopfbuchsen wird natürlich immer eine gewisse Menge an belastetem Schmieröl aus dem Kurbelwellenraum in den Kolbenunterseitenraum befördert. Ausserdem ist das im Receiverraum abgelagerte Schmieröl immer eine Mischung von abgesetztem Schmieröl, die sich aus sehr vielen vergangener Motorenzyklen zusammensetzt, so dass das im Receiverraum absetzte Schmieröl nie eine aktuelle Zusammensetzung des Schmieröls wieder gibt, wie sie der tatsächlichen aktuellen Zusammensetzung des Schmieröls im Zylinderliner entspricht.

Das heisst, das im Receiverraum abgesetzte Schmieröl enthält eine ganze Reihe von Rückständen die entweder gar nicht aus dem entsprechenden Zylinderliner stammen und / oder nicht der aktuellen Situation entsprechen. Ein weiterer gravierender Nachteil der bekannten Messmethoden liegt darin, dass die verwendeten magnetischen Messsysteme eine viel zu geringe Empfindlichkeit haben. Das führt dazu, dass verhältnismässig grosse Mengen an Schmieröl gesammelt werden müssen. Typischerweise wird das Schmieröl in Behälter gesammelt, die ein Volumen von einigen 10cm³ haben, die mehr oder weniger ganz gefüllt werden müssen, bevor eine Messung überhaupt durchführbar ist. Da wie eingangs erwähnt der Schmierölverbrauch z.B. eines modernen Grossdieselmotors sehr stark minimiert worden ist, dauert es bei den bekannten Messsystemen häufig viele Stunden bis sich in den Messgefässen eine ausreichende Menge an Schmieröl gesammelt hat, die untersucht werden kann.

Der Nachteil liegt auf der Hand. Tatsächliche Online-Messungen in dem Sinne, dass ein aktueller Verschleisszustand der Motorkomponenten ermittelt werden kann, sind überhaupt nicht möglich, da über lange Zeiten eine messbare Probe aus Schmieröl gesammelt werden muss. Das führt dazu, dass zum Beispiel ein einsetzendes Fressen des Kolbens an der Zylinderlauffläche, also das gefürchtete Scuffing, oft viel zu spät bemerkbar ist. Oft erst so spät, dass massive Schäden an den Zylinderkomponenten nicht mehr zu vermeiden sind. Die Folge ist, dass oft ein Zylinderliner ausser Betrieb gestellt und ersetzt werden muss.

Eine Aufgabe der Erfindung ist es daher, eine verbesserte Überwachungsvorrichtung, sowie ein verbessertes Überwachungsverfahren zur Überwachung eines Verschleisszustands einer Komponente einer Hubkolbenbrennkraftmaschine bereitzustellen, mit welchem zuverlässiger und schneller, bevorzugt in einem echten Online-Messverfahren, der Verschleisszustand von Komponenten der Brennkraftmaschine möglichst in Echtzeit beobachtet werden kann, so dass entsprechende Massnahmen wie zum Beispiel Wartungsmassnahmen oder eine entsprechende Anpassung der Steuerung der Maschine oder des betroffenen Zylinders ohne unnötige Zeitverzögerung vorgenommen werden kann.

Die diese Aufgaben lösenden Gegenstände der Erfindung sind durch die Merkmale der unabhängigen Ansprüche 1 und 14 gekennzeichnet.

Die abhängigen Ansprüche beziehen sich auf besonders vorteilhafte Ausführungsformen der Erfindung.

Die Erfindung betrifft somit eine Hubkolbenbrennkraftmaschine mit einer Überwachungsvorrichtung und ein Überwachungsverfahren zur Überwachung eines Verschleisszustands einer Komponente einer Hubkolbenbrennkraftmaschine umfassend einen Zylinder mit Zylinderdeckel und einer an einer Zylinderwand des Zylinders vorgesehenen Lauffläche, in welchem Zylinder ein Kolben entlang der Lauffläche zwischen einem unteren Totpunkt und einem oberen Totpunkt in einer axialen Richtung derart hin- und herbewegbar angeordnet ist, dass der Kolben, der Zylinderdeckel und die Zylinderwand im Zylinder einen Brennraum zur Verbrennung eines Gemischs aus einem Treibstoff und Luft bilden, wobei eine Ölsammeleinrichtung zum Sammeln von Schmieröl aus dem Zylinder vorgesehen ist, so dass eine vorgebbare Messmenge von Schmieröl aus dem Zylinder einer Messeinrichtung zuführbar ist und wobei die Ölsammeleinrichtung eine an der Zylinderwand zwischen dem unteren Totpunkt des Kolbens und dem Zylinderdeckel vorgesehene Ölsammelöffnung umfasst. Erfindungsgemäss wird die Messmenge an Schmieröl unmittelbar von der Lauffläche des Zylinders und unmittelbar aus einem Kolbenringpaket des Kolbens der Sammeleinrichtung zugeführt.

Dadurch, dass die Messmenge an Schmieröl nicht mehr aus im Receiverraum abgesetztem und angesammeltem Schmieröl besteht, sondern nur Schmieröl zur Messung verwendet wird, dass unmittelbar und aktuell aus dem Zylinderraum stammt und damit nicht mit Fremdstoffen kontaminiert ist, enthält das erfindungsgemäss gesammelte Schmieröl im wesentlichen nur noch Informationen, die den aktuellen Verschleisszustand der Zylinderkomponenten wie Kolben, Kolberinge, Zylinderlauffläche, Gaswechselventil usw. widerspiegeln.

Damit ist es erstmals möglich, den tatsächlichen Verschleisszustand der Motorkomponenten zu überwachen, ohne störende Fremdeinflüsse, wie zum Beispiel Verschmutzungen, die nicht aus dem Zylinderliner direkt stammen oder nicht mit dem Zeitpunkt der Messung korrelieren.

Um möglichst Echtzeitmessungen durchführen zu können, wird wie in den Ansprüchen offenbart bevorzugt eine schnelle Messmethode verwendet. Die erfindungsgemässe schnelle Messmethode eignet sich im besonderen auch dafür, dass Messungen auch am sich im transienten Betriebszustand befindlichen Motor durchgeführt werden können, wobei sich der transiente Betriebszustand auf eine während der Messung veränderlichen Motordrehzahl oder auf während der Messung veränderlichen Motorbelastung oder auf eine Kombination von während der Messung veränderlicher Motordrehzahl und Motorbelastung bezieht.

Ein besonders bevorzugtes Ausführungsbeispiel einer schnellen Messmethode, die zu dem auch aus apparativer Hinsicht kostengünstig und einfach ist, und die vor allem nur sehr kleine Mengen an Schmieröl für eine Messung benötigt, bedient sich im wesentlichen eines Messkondensators, der geometrisch so ausgelegt ist, dass die während eines einzigen, oder zumindest während einiger weniger Verbrennungszyklen gesammelten Menge an Schmieröl ausreicht, um den Kondensator mit einer messbaren Menge an Schmieröl zu füllen.

Zum Beispiel wird ein Kondensator, z.B. ein Plattenkondensator mit einem kleinen Plattenabstand verwendet.

Sehr vorteilhaft kann auch ein zylindrischer Plattenkondensator verwendet werden, der aus zwei spiralartig ineinander gewickelten Metallflächen besteht, die möglichst nah zueinander angeordnet werden.

Das gesammelte Schmieröl kann dann zur Messung einfach in den zylindrischen Plattenkondensator eingeblasen werden und eine Messung durchgeführt werden. Nach Abschluss der Messung kann der zylindrische Kondensator zum Beispiel mit Druckluft gereinigt werden, so dass in einem nächsten Zyklus für eine weitere Messung neu gesammeltes Schmieröl in den zylindrischen Kondensator eingeblasen werden kann.

Mit einer an sich bekannten Messbrücke kann dann zum Beispiel der komplexe Leitwert, bevorzugt noch frequenz- und / oder temperaturabhängig, oder in Abhängigkeit von der angelegten elektrischen Spannung, der Temperatur oder der Variation anderer relevanter Parameter gemessen werden. Aus den Messwerten kann dann zum Beispiel durch Vergleich mit Eichmessungen oder durch die Auswertung geeigneter mathematischer Funktionen direkt auf Verunreinigungen wie Wasser, Metalle, wie Eisen, Chrom, Vanadium oder anderer Metalle geschlossen werden. Auch auf chemische Verunreinigungen kann geschlossen werden, vor allem auf Ionen bildende Verunreinigungen oder Komponenten wie Säuren oder Laugen usw., so dass auch Veränderungen in der chemischen Zusammensetzung zugänglich sind, und zum Beispiel Element Konzentrationen von Schwefel, Phosphor oder anderer chemischer Elemente oder Komponenten festgestellt werden können.

Zur Erhöhung der Messgenauigkeit und damit nicht zuletzt zur Herabsetzung des notwendigen Messvolumens an Schmieröl, kann der Kondensator in an sich bekannter Weise zum Beispiel in einen elektrischen Stromkreis eingesetzt werden, der neben anderen elektrischen Komponenten noch eine Spule vorgegebener Induktivität enthält, und so einen Schwingkreis von vorgegebener Resonanzfrequenz bildet.

Solche Anordnungen reagieren auf eine Änderung der Induktivität der Spule oder der Dielektrizitätskonstanten des Kondensators extrem empfindlich mit einer Änderung der Resonanzfrequenz. Damit steht durch die vorliegende Erfindung mit einer solchen Anordnung, wobei auch mehrere Schwingkreise unterschiedlicher Resonanzfrequenz gekoppelt werden können, ein extrem empfindliches Messinstrument zur Verfügung, mit dem auch mit einem sehr kleinen Probevolumen eine sehr hohe Messgenauigkeit, auch in Bezug auf verschiedene Inhaltsstoffe erzielbar ist, zur Verfügung.

Dabei versteht es sich, dass das Schmieröl auch in eine geeignet konstruierte Spule eingefüllt werden kann, was in Abhängigkeit von der Verschmutzung zur Veränderung der Induktivität der Spule führt. Auch kann natürlich ein entsprechend präparierter Kondensator oder eine entsprechend präparierte Spule in einem Messkreis kombiniert werden.

Darüber hinaus können auch andere Grössen als z.B. die Messfrequenz oder die Messamplitude geeignet verwendet werden, um z.B. die Messgenauigkeit zu erhöhen oder um bestimmte Phänomene, wie zum Beispiel Resonanz Erscheinungen auszunutzen. So kann beispielweise am Messkörper, also zum Beispiel am Öl gefüllten Kondensator, eine elektrische oder eine anders betriebene Heizung vorgesehen werden, so dass eine Messung bei einer definierten Temperatur durchgeführt werden kann, bei der beispielsweise ein bestimmter Relaxationsprozess besonders gut gemessen werden kann. Dem Fachmann sind entsprechende weitere Manipulationsmethoden bekannt, durch deren Einsatz interessierende Grössen besonders gut bestimmt werden können.

Es versteht sich, dass zur Verbesserung der Messgenauigkeit zum Beispiel auch andere Grössen mit geeigneten, an sich bekannten Sensoren erfasst werden können. So können zum Beispiel Sensoren vorgesehen werden, die die Temperatur bei der Messung überwachen, oder automatisch die Menge an Schmieröl überwachen, die für eine aktuelle Messung zur Verfügung steht, so dass gegebenenfalls die direkte Messgrösse entsprechend korrigiert werden kann.

Gesammelt wird das Schmieröl bevorzugt durch geeignete Öffnungen in der Zylinderwand, so dass orts- und / oder zeitabhängige Proben entnommen werden können. Das kann z.B. dadurch geschehen, dass das Schmieröl auf Grund des im Zylinder und / oder Ringpaket in Bezug auf die Umgebung herrschenden Überdrucks durch die Öffnungen in der Zylinderwand einem Sammel- und / oder Messsystem zugeführt wird. Der erwähnte, im Ringpaket in Bezug auf die Umgebung herrschende Überdruck ergibt sich sowohl bei Hubkolben-Motoren nach Zweitakt-Bauweise als auch nach Viertakt-Bauweise, so dass das erfindungemässe Messprinzip sowohl bei Zweitakt- als auch bei Viertakt-Hubkolben-Motoren angewendet werden kann.

Aber auch eine Entnahme von Schmieröl über die Spülschlitze ist vorteilhaft möglich. Wenn der Kolben auf seiner Bewegung in Richtung zum unteren Totpunkt die Spülschlitze, die in den Receiverraum münden, passiert, wird das Schmieröl aus den Kolbenringnuten bzw. aus dem Kolbenringpaket, in dem eine bestimmte Menge an Schmieröl immer gespeichert ist, durch den im Kolbenringpaket herrschenden Überduck in Form einer Schmierölwolke in den Receiverraum geblasen, wo dann durch eine geeignete Sammeleinrichtung das Schmieröl aus der Schmierölwolke aufgesammelt werden kann.

Wie dem Fachmann bekannt ist, läuft der Gasdruck wie in einer Labyrinthdichtung durch das Kolbenringpaket und wird dort gemeinsam mit dem sich im Kolbenringpaket befindenden Schmieröl gespeichert und kann, solange das Kolbenringpaket dichtend an der Lauffläche der Zylinderwand anliegt, praktisch nicht entweichen. Passiert der Kolben auf seiner Dekompressionsbewegung in Richtung zum unteren Totpunkt UT dann die Spülschlitze, so kann das im Kolbenringpaket gespeicherte Gas zusammen mit dem im Kolbenringpaket gespeicherten Schmieröl schlagartig über die Spülschlitze in den Receiverraum in Form einer Schmierölwolke entweichen, wodurch der Receiverraum natürlich massiv mit Schmieröl kontaminiert wird.

Diese an sich eher negative Begleiterscheinung kann somit positiv durch die Erfindung ausgenützt werden.

Bei nicht aufgeladenen Hubkolben-Motoren nach Zweitakt- und Viertakt-Bauart kann aber der Zustand auftreten, dass am Ende des auf die Verbrennung folgenden Expansionstakts der Gasdruck im Kolbenringpaket nicht mehr wesentlich über dem Umgebungsdruck liegt und somit mit der erfindungsgemässen Ölsammeleinrichtung keine genügend grosse Ölmenge erwachsen würde. Diesem Umstand kann abgeholfen werden, indem auf der Abluftseite des verwendeten ÖI-/Luft-Separators ein von aussen künstlich, z.B. durch ein Gebläse hergestellter Unterdruck aufgebracht wird. Dadurch ergibt sich wieder ein genügend grosser Druckunterschied zwischen Ringpaket und Abluftseite des verwendeten ÖI-/Luft-Separators, womit die Funktion des Ölsammel- und Messsystems auch bei nicht aufgeladenen Hubkolben-Motoren sichergestellt werden kann.

Erfindungsgemäss umfasst die Ölsammeleinrichtung eine an der Zylinderwand zwischen dem unteren Totpunkt des Kolbens und dem Zylinderdeckel vorgesehene Ölsammelöffnung. Im Speziellen kann dabei die Ölsammeleinrichtung ein von einer Steuereinrichtung betätigbares Ölsammelventil umfassen.

Die Hubkolbenbrennkraftmaschine kann dabei sowohl eine längs gespülte Brennkraftmaschine mit Spülschlitzen, insbesondere ein langsam laufender Zweitakt-Grossdieselmotor sein, wobei die Ölsammelöffnung bevorzugt derart im Bereich der Spülschlitze angeordnet sind, dass über die Spülschlitze austretendes Schmieröl durch die Ölsammelöffnung sammelbar ist. Es versteht sich dabei von selbst, dass die Hubkolbenbrennkraftmaschine auch eine Berennkraftmaschine sein kann, die nach dem Viertakt-Prinzip betrieben wird.

Wenn die Hubkolbenbrennkraftmaschine eine Viertakt-Hubkolbenbrennkraftmaschine ist, sind die Ölsammelöffnung bevorzugt derart am Zylinder zwischen der Position einer Krone des Kolbens im oberen Totpunkt und der Position einer Kolbenunterseite im unteren Totpunkt angeordnet ist, dass Schmieröl aus dem Zylinder durch die Ölsammelöffnung sammelbar ist. Besonders bevorzugt liegen geeignete Öffnungen der Ölsammelvorrichtung am Zylinder zwischen der Position der Kolbenkrone im oberen Totpunkt und des untersten Ringes des Kolberingpakets des Kolbens im unteren Totpunkt. Durch eine derartige Anordnung der Öffnungen der Ölsammelvorrichtung an der Zylinderwand kann insbesondere sichergestellt werden, dass Schmieröl im wesentlichen von der gesamten, vom Schmieröl relevant benetzte Lauffläche der Kolbenringe eines Viertaktmotors durch die Ölsammelvorrichtung sammelbar ist.

Besonders bevorzugt umfasst die Ölsammeleinrichtung einen Ölsammelraum, der vorteilhaft ein integraler Bestandteil der Zylinderwand sein kann, wobei im Ölsammelraum gesammeltes Schmieröl der Messeinrichtung über die Ölsammelöffnung zuführbar ist.

Häufig sind mindestens zwei Ölsammelöffnungen vorgesehen, die in Umfangsrichtung und / oder in Bezug auf die axiale Richtung zueinander versetzt am Zylinder angeordnet sind, wobei die Ölsammelöffnung in einem speziellen Ausführungsbeispiel derart ausgestaltet ist, dass ein Schmieröldurchsatz einstellbar ist.

Das Messsystem umfasst dabei vorteilhaft eine elektromagnetische Messeinheit, insbesondere eine Messeinheit zur amplitudenabhängigen und / oder frequenzabhängigen und / oder frequenzunabhängigen Bestimmung einer Kapazität, einer magnetischen Permeabilität, einer elektrischen Gleichstromleitfähigkeit und / oder Wechselstromleitfähigkeit, und / oder einer komplexen elektrischen Leitfähigkeit und / oder eines komplexen elektrischen Widerstands der gesammelten Messmenge von Schmieröl, wobei die Überwachungsvorrichtung bevorzugt miniaturisiert direkt in der Zylinderwand vorgesehen sein kann.

In einem anderen Ausführungsbeispiel der vorliegenden Erfindung umfasst das Messsystem eine Röntgenmesseinheit, insbesondere zur Bestimmung einer Transmissionseigenschaft und / oder einer Absorptionseigenschaft und / oder einer Reflexionseigenschaft und / oder einer Fluoreszenzeigenschaft der gesammelten Messmenge von Schmieröl.

In einem weiteren Ausführungsbeispiel umfasst das Messsystem eine optische Messeinheit zur Bestimmung einer optischen Transmissionseigenschaft und / oder einer optischen Absorptionseigenschaft und / oder einer optischen Reflexionseigenschaft und / oder einer optischen Fluoreszenzeigenschaft der gesammelten Messmenge von Schmieröl, wobei die optische Messeinheit bevorzugt eine Infrarot Messeinheit und / oder eine Ultraviolett Messeinrichtung ist.

In der Praxis kann das Messsystem insbesondere eine chemische Messeinheit zur Bestimmung einer chemischen Zusammensetzung der gesammelten Messmenge von Schmieröl umfassen, wobei das Messsystem ein Messsystem zur Bestimmung eines Gehaltes an Wasser und / oder eines Gehaltes an Metall, insbesondere Eisen und / oder Chrom und / oder Vanadium, und / oder zur Bestimmung eines Gehaltes an Phosphor und / oder Schwefel der gesammelten Messmenge von Schmieröl umfasst.

Die Erfindung betrifft weiterhin eine Hubkolbenbrennkraftmaschine, insbesondere einen Zweitakt-Grossdieselmotor oder einen Viertakt-Motor mit einer erfindungsgemässen Überwachungsvorrichtung, sowie ein Überwachungsverfahren zur Überwachung eines Verschleisszustands einer Komponente einer Hubkolbenbrennkraftmaschine.

Das erfindungsgemässe Überwachungsverfahren betrifft ein Überwachungsverfahren zur Überwachung einer Hubkolbenbrennkraftmaschine umfassend einen Zylinder mit Zylinderdeckel und einer an einer Zylinderwand des Zylinders vorgesehenen Lauffläche, in welchem Zylinder ein Kolben entlang der Lauffläche zwischen einem unteren Totpunkt und einem oberen Totpunkt in einer axialen Richtung derart hin- und herbewegbar angeordnet ist, dass der Kolben, der Zylinderdeckel und die Zylinderwand im Zylinder einen Brennraum zur Verbrennung eines Gemischs aus einem Treibstoff und Luft bilden. Dabei wird eine Ölsammeleinrichtung zum Sammeln von Schmieröl aus dem Zylinder vorgesehen, und im Betriebszustand eine vorgebbare Messmenge von Schmieröl aus dem Zylinder einer Messeinrichtung zugeführt. Erfindungsgemäss wird die Messmenge an Schmieröl unmittelbar von der Lauffläche des Zylinders und / oder unmittelbar aus dem Brennraum und / oder unmittelbar aus einem Kolbenringpaket des Kolbens der Sammeleinrichtung zugeführt.

Zur Durchführung des erfindungsgemässen Überwachungsverfahrens umfasst die Ölsammeleinrichtung bevorzugt eine an der Zylinderwand zwischen dem unteren Totpunkt des Kolbens und dem Zylinderdeckel vorgesehene Ölsammelöffnung.

Im Speziellen kann die Ölsammeleinrichtung ein von einer Steuereinrichtung betätigbares Ölsammelventil umfassen, so dass im Betriebszustand eine vorgegebene Menge an Schmieröl aus dem Zylinder abgeführt werden kann.

Die Hubkolbenbrennkraftmaschine ist dabei zum Beispiel eine längs gespülte Brennkraftmaschine mit Spülschlitzen, insbesondere ein langsam laufender Zweitakt-Grossdieselmotor ist, und die Ölsammelöffnung wird derart im Bereich der Spülschlitze angeordnet, dass über die Spülschlitze austretendes Schmieröl durch die Ölsammelöffnung im Betriebszustand gesammelt wird. Es versteht sich, dass das erfindungsgemässe Überwachungsverfahren natürlich auch erfolgreich bei einer Brennkraftmaschine eingesetzt werden kann, die nach dem Viertakt-Prinzip betrieben wird.

Besonders bevorzugt umfasst die Ölsammeleinrichtung zur Durchführung des erfindungsgemässen Überwachungsverfahrens einen Ölsammelraum, der bevorzugt ein integraler Bestandteil der Zylinderwand ist, so dass im Ölsammelraum gesammeltes Schmieröl der Messeinrichtung zuführbar ist, in im Speziellen auch über die Ölsammelöffnung zugeführt werden kann.

In der Praxis werden meist mindestens zwei Ölsammelöffnungen vorgesehen, die in Umfangsrichtung und / oder in Bezug auf die axiale Richtung zueinander versetzt am Zylinder angeordnet werden, so dass an verschiedenen Orten im Zylinder Proben von Schmieröl entnommen werden können, wobei die Ölsammelöffnung bevorzugt derart ausgestaltet wird, dass ein Schmieröldurchsatz eingestellt, vorteilhaft automatisch eingestellt werden kann.

Zur Durchführung des erfindungsgemässen Überwachungsverfahrens umfasst das Messsystem besonders bevorzugt eine elektromagnetische Messeinheit, so dass insbesondere eine amplitudenabhängige Bestimmung und / oder frequenzabhängige Bestimmung und / oder eine frequenzunabhängige Bestimmung einer Kapazität, einer magnetischen Permeabilität, einer elektrischen Gleichstromleitfähigkeit und / oder einer Wechselstromleitfähigkeit, und / oder einer komplexen elektrischen Leitfähigkeit und / oder eines komplexen elektrischen Widerstands der gesammelten Messmenge von Schmieröl durchgeführt werden.

In einem anderen Ausführungsbeispiel kann das Messsystem eine Röntgenmesseinheit umfassen, so dass insbesondere eine Bestimmung einer Transmissionseigenschaft und / oder einer Absorptionseigenschaft und / oder einer Reflexionseigenschaft und / oder einer Fluoreszenzeigenschaft der gesammelten Messmenge von Schmieröl durchgeführt werden kann.

In einem weiteren speziellen Ausführungsbeispiel kann das Messsystem eine optische Messeinheit umfassen, so dass insbesondere eine Bestimmung einer optischen Transmissionseigenschaft und / oder einer optischen Absorptionseigenschaft und / oder einer optischen Reflexionseigenschaft und / oder einer optischen Fluoreszenzeigenschaft der gesammelten Messmenge von Schmieröl durchgeführt werden kann, wobei als optische Messeinheit bevorzugt eine Infrarot Messeinheit und / oder eine Ultraviolett Messeinrichtung verwendet wird.

In der Praxis wird das Messsystem häufig eine chemische Messeinheit umfassen, so dass eine Bestimmung einer chemischen Zusammensetzung der gesammelten Messmenge von Schmieröl durchgeführt werden kann, wobei mit dem Messsystem besonders bevorzugt ein Gehalt an Wasser und / oder ein Gehalt an Metall, insbesondere Eisen und / oder Chrom und / oder Vanadium, und / oder ein Gehalt an Phosphor und / oder Schwefel der gesammelten Messmenge bestimmt wird.

Die mit dem Messsystem erfassten Daten werden insbesondere mittels einer Look-up Tabelle und / oder mittels einer vorgegebenen mathematischen Funktion und / oder mittels einer Eichung ausgewertet, wobei im Speziellen daraus ein Verschleisszustand einer Komponente der Hubkolbenbrennkraftmaschine, insbesondere der Verschleisszustand des Kolbens und / oder eines Kolbenrings und / oder der Lauffläche der Zylinderwand, und / oder eines Gaswechselventils und / oder einer anderen Motorkomponente bestimmt wird.

Besonders bevorzugt wird bei jedem Motorzyklus eine Auswertung der Messung an der gesammelten Messmenge von Schmieröl vorgenommen wobei besonders vorteilhaft während einer vorgegeben Anzahl von Motorzyklen eine vorgegebene Messmenge an Schmieröl gesammelt wird, und eine Messung an der vorgegebenen Messmenge an Schmieröl durchgeführt und ausgewertet wird.

Durch eine Auswertung der gesammelten Messdaten kann zum Beispiel ein Wartungszeitpunkt für die Wartung einer vorgegebenen Motorkomponente, bevorzugt auch automatisch ermittelt werden.

Auch kann die Hubkolbenbrennkraftmaschine selbstverständlich in Abhängigkeit von einem Messergebnis an der gesammelten Messmenge von Schmieröl gesteuert und / oder geregelt werden.

Bei einem speziellen Ausführungsbeispiel der vorliegenden Erfindung, das besonders wichtig für die Praxis ist, handelt es sich um ein "selbst regulierendes Schmiersystem", also im wesentlichen um ein elektronisch geregeltes Schmiersystem, das sehr homogene und leistungsfähige Eigenschaften des Ölfilms garantiert, gleichzeitig die Belastungen auf den Ölfilm reduziert und einen Abbau des Ölfilms sowohl in Umfangsrichtung als auch in axialer Richtung des Zylinderliners verhindert, und zwar unter allen Betriebsbedingungen.

Die vorliegende Erfindung fokussiert damit auf die ständige neu Justierung der Betriebsparameter des Schmiersystems, wie zum Beispiel die Schmierölförderrate, die vertikale und / oder horizontale Verteilung des Schmieröls, die Einspritzfrequenz, usw., falls die entsprechenden Schmierfilmeigenschaften nicht mehr mit den vorgegebenen Parameterwerten übereinstimmen oder unterhalb oder oberhalb vorgegebener Parametergrenzen liegen. Darüber hinaus ist die Qualität bzw. Leistungsfähigkeit der Schmierung und des Schmierölfilms abhängig von den Betriebsbedingungen unter denen die Brennkraftmaschine betrieben wird, wie zum Beispiel die Last, die Feuchtigkeit der Spülluft, der Schwefelgehalt im Treibstoff, die Treibstoffqualität, der Qualität des Schieröls usw.

Werden zum Beispiel zwei oder mehrere in axialer- und / oder in Umfangsrichtung voneinander beabstandete Ölsammelöffnungen am Zylinder vorgesehen, so ist es möglich, Schmieröl zum Beispiel direkt aus dem Kolbenringpaket oder von der Lauffläche des Zylinderliners an verschiedenen Stellen des Zylinders zu sammeln, mit einer erfindungsgemässen Messeinrichtung zu analysieren und daraus eine Art "Landkarte" der Verteilung der Eigenschaften des Schmierölfilms über die gesamte Lauffläche des Zylinders zu erstellen. Aus den so gewonnenen Messdaten kann dann über eine geeignete Steuerung und / oder Regelung die Zufuhr von Schmieröl auf die Lauffläche des Zylinders zeitabhängig und / oder ortsabhängig derart gesteuert und / oder geregelt werden, dass eine gleichmässige Leistungsfähigkeit und Qualität des Schmierölfilms über die gesamte Lauffläche des Zylinders dauerhaft und automatisch garantiert ist.

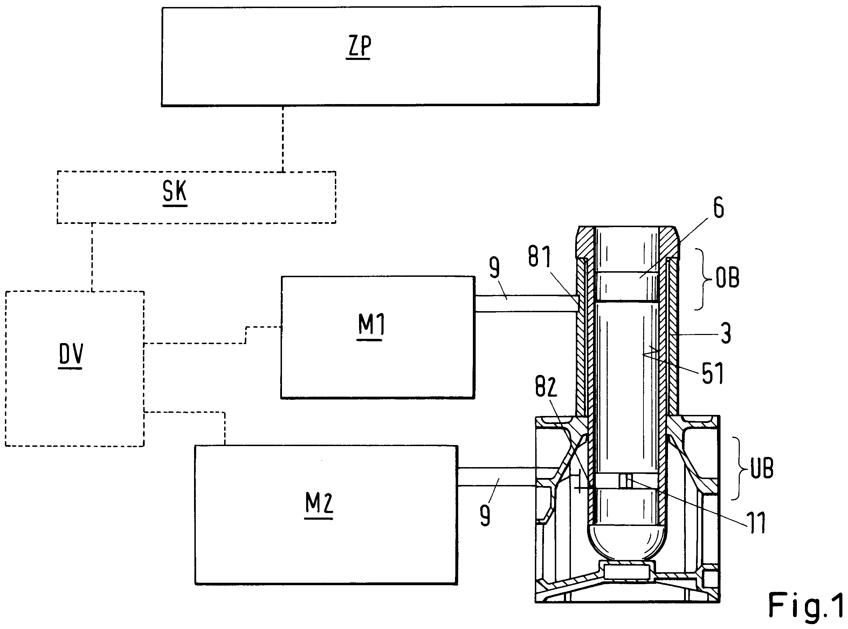

Wie anhand von Fig. 1 schematisch gezeigt, wird bevorzugt aus einem oberen Bereich OB an einer ersten Ölsammelöffnung 81 und einem unteren Bereich UB an einer zweiten Ölsammelöffnung 81 eines Zylinders 3 Schmieröl 9 getrennt gesammelt und die Eigenschaften des so gesammelten Schmieröls 9 wie z.B. Alkalinität (BN-Wert), der Eisenghalt, der Wassergehalt usw. getrennt in den beiden Messeinrichtungen M1 und M2 analysiert und einer Datenverarbeitungsanlage DV mit Datenerfassungseinrichtung zugeführt. Im vorliegenden Beispiel liegt die untere zweite Ölsammelöffnung 81 im Bereich der Spülschlitze 11, so dass durch die untere zweite Ölsammelöffnung 81 Schmieröl 9 aus dem Kolbenringpaket gesammelt wird, während die obere erste Ölsammelöffnung Schmieröl direkt von der Lauffläche des Zylinders 3 sammelt.

In an sich bekannter Weise hat der Zylinder 3 der Fig. 1 dabei mehrere Schmieröldüsen, die aus Übersichtgründen in Fig. 1 nicht dargestellt sind, mit denen frisches Schmieröl nach einem vorgebbaren Schema auf die Lauffläche 51 des Zylinders 3 aufgetragen werden kann. Die mehreren Schmieröldüsen sind dabei sowohl in Umfangsrichtung als auch in vertikaler Richtung voneinander beabstandet am Zylinder 3 vorgesehen.

Die Datenverarbeitungsanlage DV bestimmt aus den aus dem Kolbenringpaket des Kolbens 6 und dem Zylinderliner 3 getrennt gemessenen Eigenschaften des Schmieröls 9 in Abhängigkeit vom Ort im Zylinderliner 3 neue Zuführparameter ZP für das Schmieröl, wie zum Beispiel Schmierölmenge, Frequenz der Zuführung des Schmieröls in den Zylinder usw., wodurch eine Abweichung des Schmierölfilms von den vorgegebenen Sollwert Parametern wieder ausgeglichen wird, so dass durch die Erfindung der Schmierölfilm zu jeder Zeit und an jedem Ort auf der Lauffläche 51 des Zylinders 3 optimierbar ist. Die so neu ermittelten neuen Zuführparameter ZP werden an einen Schmierölkontroller SK weiter gegeben, der die weitere Zufuhr des Schmieröls 9 über die mehreren Schmieröldüsen so einstellt, dass der Schmierölfilm auf der gesamten Lauffläche 51 des Zylinders 3 wie zuvor beschrieben optimiert wird.

Wesentlich bei dem speziellen Ausführungsbeispiel gemäss Fig. 1 ist, dass Schmieröl 9 aus dem oberen Bereich OB des Zylinders 3 und aus dem unteren Bereich UB des Zylinders 3 getrennt gesammelt und analysiert wird. Das Schmieröl 9 das im oberen Bereich OB des Zylinders 3 gesammelt wird, stammt zum Beispiel direkt von der Lauffläche 51. Das kann dadurch sicher gestellt werden, dass die Ölsammelöffnung 81 im oberen Bereich OB des Zylinders 3 dann eine Probe an Schmieröl 9 entnimmt, wenn der Kolben 6 die obere Ölsammelöffnung 81 nicht passiert. Die untere Ölsammelöffnung 81 an den Spülschlitzen 11 sammelt dagegen genau dann eine Probe an Schmieröl 9, wenn der Kolben 6 die Spülschlitze 11 passiert. Dadurch stammt die Probe an Schmieröl 9, die im unteren Bereich UB des Zylinders 3 gesammelt wird direkt aus dem Kolbenringpaket des Kolbens 6, da sich der Überdruck im Kolbenringpaket durch die Spülschlitze 11 entlädt und so im Kolbenringpaket gesammeltes Schmieröl 9 aus dem Kolbenringpaket ausgeblasen wird, so dass aus dem aus dem Kolbenringpaket ausgeblasenen Schmieröl 9 dann durch die Ölsammelöffnung 81 im unteren Bereich UB eine Probe an Schmieröl 9 entnommen werden kann.

Soll beispielweise der BN-Wert des Schmieröls im Zylinder optimiert werden, kann das folgendermassen erreicht werden. Der BN-Wert des Schmieröls ist, wie dem Fachmann wohl bekannt ist, ein Mass für das alkalische Verhalten des Schmieröls, also ein Mass für dessen Alkalinität. Das Schmieröl muss dabei eine gewisse Alkalinität aufweisen, da im Zylinder zum Beispiel durch den Verbrennungsprozess sehr aggressive Säuren entstehen können, die möglichst neutralisiert werden müssen, damit diese Säuren die einzelnen Komponenten im Zylinder, wie Kolben, Lauffläche, Kolbenringe, Auslassventil usw. möglichst nicht angreifen können.

Daher wird ein minimaler BN-Wert des Schmieröls im Zylinder gefordert, der von dem konkreten Motor, den Betriebsbedingen und anderen Betriebsparametern abhängen kann.

Bei dem erfindungsgemässen Überwachungsverfahren wird dabei in einem speziellen Ausführungsbeispiel zunächst der BN-Wert zum Beispiel für das im Kolbenringpaket gesammelte Schmieröl auf einen vorgegebenen minimalen BN-Wert geregelt, der zum Beispiel den vorgegebenen Sollwert BN=5 haben kann. Dieser vorgegebene Sollwert BN=5 kann natürlich in einem anderen Motor oder bei anderen Betriebsbedingungen einen ganz anderen Wert als BN=5 haben.

Dabei soll der vorgegeben minimale Sollwert von zum Beispiel BN=5 möglichst nicht wesentlich überschritten werden, da der Sollwert auch ein Mass für die Menge an zugeführtem Schmieröl ist, die es allein schon aus Kostengründen gleichzeitig zu optimieren bzw. zu minimieren gilt. Da das im Kolbenring befindliche Schmieröl beim Expansionstakt über die gesamte Höhe des Zylinderliners von der Lauffläche aufgesammelt wurde, stellt der BN-Wert des aus dem Kolbenringpaket stammenden Schmieröls einen gewissen Mittelwert einer Verteilung des BN-Werts über die Höhe der Lauffläche in der axialen Richtung dar.

Daher der kann der BN-Wert über die Höhe des Zylinderliners in der vertikalen Richtung entlang der Zylinderachse dadurch im wesentlichen gleichmässig eingestellt werden, dass versucht wird, den BN-Wert des Schmieröls, das aus dem oberen Bereich des Zylinders stammt, möglichst identisch zu dem BN-Wert des Schmieröls einzustellen, das aus dem Kolbenringpaket im Bereich der Spülschlitze ausgeblasen wird. Idealerweise ist der BN-Wert des Schmieröls aus dem oberen Bereich des Zylinders gleich dem BN-Wert des Schmieröls aus dem Kolbenringpaket, wie er im Bereich der Spülschlitze gemessen wird. In dem Fall wird der BN-Wert entlang der Zylinderachse überall im wesentlichen gleich sein.

Die Regulierung des BN-Werts erfolgt dabei durch die Einstellung der Zuführparameter, also durch die Regulierung der Zuführung des Schmieröls über die Schmieröldüsen, die bevorzugt einzeln oder in bestimmten Gruppen jeweils separat ansteuerbar sind.

Es versteht sich, dass dadurch dass auch in Umfangrichtung des Zylinderliners mehrere Ölsammelöffnungen vorgesehen sein können, der BN-Wert auch in Umfangsrichtung gleichmässig also homogen eingestellt werden kann.

Somit ist es durch die Erfindung erstmals möglich, dass sowohl in Umfangsrichtung als auch in der vertikalen Richtung der BN-Wert des Schmieröls auf der gesamten Lauffläche des Zylinders gleichmässig auf im wesentlichen denselben Wert einstellbar ist, wobei zusätzlich eine Minimierung bzw. Optimierung des Schmierölverbrauchs erreicht wird, da gleichzeitig auch auf einen vorgegebenen optimalen BN-Wert, von z.B. BN=5 geregelt wird.

Es versteht sich von selbst, dass völlig analog auch andere Parameter des Schmieröls in analoger Weise durch die Erfindung homogenen über die Lauffläche und auf einen vorgegebenen Wert optimierbar sind.

Ausserdem ist dem Fachmann sofort klar, dass die oben beschrieben Schritte des Optimierungsverfahrens nur beispielhaft zu verstehen sind und insbesondere die Schritte auch in einer anderen Reihenfolge erfolgen können, zusätzliche andere Schritte eingefügt können sein, oder in anderen einfachen Fällen bestimmte Optimierungsschritte auch fehlen können.

Die Erfindung betrifft weiterhin ein Messsystem zur Bestimmung einer Zusammensetzung eines Fluids, insbesondere eines Öl, im Speziellen eines Schmieröls oder eines Treibstoffs für eine Hubkolbenbrennkraftmaschine. Erfindungsgemäss umfasst das Messsystem dabei eine elektromagnetische Messeinheit, insbesondere eine Messeinheit zur amplitudenabhängigen und / oder frequenzabhängigen und / oder frequenzunabhängigen Bestimmung einer Kapazität, einer magnetischen Permeabilität, einer elektrischen Gleichstromleitfähigkeit und / oder Wechselstromleitfähigkeit, und / oder einer komplexen elektrischen Leitfähigkeit und / oder eines komplexen elektrischen Widerstands einer vorgebbaren Messmenge des Fluids umfasst.

In einem anderen Ausführungsbeispiel kann das Messsystem eine Röntgenmesseinheit umfassen, insbesondere zur Bestimmung einer Transmissionseigenschaft und / oder einer Absorptionseigenschaft und / oder einer Reflexionseigenschaft und / oder einer Fluoreszenzeigenschaft der Messmenge von Fluid.

In einem weiteren Ausführungsbeispiel kann das Messsystem eine optische Messeinheit zur Bestimmung einer optischen Transmissionseigenschaft und / oder einer optischen Absorptionseigenschaft und / oder einer optischen Reflexionseigenschaft und / oder einer optischen Fluoreszenzeigenschaft Eigenschaft der Messmenge von Fluid umfassen, wobei die optische Messeinheit bevorzugt eine Infrarot Messeinheit und / oder eine Ultraviolett Messeinrichtung ist.

In der Praxis wird das Messsystem häufig eine chemische Messeinheit zur Bestimmung einer chemischen Zusammensetzung der Messmenge von Fluid umfassen, wobei in einem speziellen Ausführungsbeispiel das Messsystem ein Messsystem zur Bestimmung eines Gehaltes an Wasser und / oder eines Gehaltes an Metall, insbesondere Eisen und / oder Chrom und / oder Vanadium, und / oder ein Messsystem zur Bestimmung eines Gehaltes an Phosphor und / oder Schwefel in der Messmenge von Fluid ist.

Die Erfindung betrifft darüber hinaus ein Messverfahren zur Bestimmung einer Zusammensetzung eines Fluids, insbesondere eines Öl, im Speziellen eines Schmieröls oder eines Treibstoffs für eine Hubkolbenbrennkraftmaschine. Erfindungsgemäss wird dabei ein Messsystem verwendet, welches Messsystem eine elektromagnetische Messeinheit umfasst, so dass insbesondere eine amplitudenabhängige Bestimmung und / oder frequenzabhängige Bestimmung und / oder eine frequenzunabhängige Bestimmung einer Kapazität, einer magnetischen Permeabilität, einer elektrischen Gleichstromleitfähigkeit und / oder einer Wechselstromleitfähigkeit, und / oder einer komplexen elektrischen Leitfähigkeit und / oder eines komplexen elektrischen Widerstands der gesammelten Messmenge von Schmieröl durchgeführt werden kann.

Bei einem anderen Ausführungsbeispiel eines erfindungsgemässen Messverfahrens umfasst das Messsystem als elektromagnetische Messeinheit eine Röntgenmesseinheit, so dass insbesondere eine Bestimmung einer Transmissionseigenschaft und / oder einer Absorptionseigenschaft und / oder einer Reflexionseigenschaft und / oder einer Fluoreszenzeigenschaft der Messmenge von Fluid durchgeführt werden kann.

Bei einem weiteren Ausführungsbeispiel umfasst das Messsystem als elektromagnetische Messeinheit eine optische Messeinheit, so dass insbesondere eine Bestimmung einer optischen Transmissionseigenschaft und / oder einer optischen Absorptionseigenschaft und / oder einer optischen Reflexionseigenschaft und / oder einer optischen Fluoreszenzeigenschaft der Messmenge von Fluid durchgeführt werden kann, wobei als optische Messeinheit bevorzugt eine Infrarot Messeinheit und / oder eine Ultraviolett Messeinrichtung verwendet wird.

In einem für die Praxis wichtigen Ausführungsbeispiel umfasst das Messsystem eine chemische Messeinheit so dass eine Bestimmung einer chemischen Zusammensetzung der Messmenge von Fluid durchgeführt werden kann, wobei mit dem Messsystem besonders bevorzugt ein Gehalt an Wasser und / oder ein Gehalt an Metall, insbesondere Eisen und / oder Chrom und / oder Vanadium, und / oder ein Gehalt an Phosphor und / oder Schwefel der Messmenge von Fluid bestimmt wird.

Im Speziellen werden die mit dem Messsystem erfassten Daten, insbesondere mittels einer Look-up Tabelle und / oder mittels einer vorgegebenen mathematischen Funktion und / oder mittels einer Eichung ausgewertet und daraus ein Verschleisszustand einer Komponente der Hubkolbenbrennkraftmaschine, insbesondere der Verschleisszustand des Kolbens und / oder eines Kolbenrings und / oder der Lauffläche der Zylinderwand, und / oder eines Gaswechselventils und / oder einer anderen Motorkomponente bestimmt.

In einem bevorzugten Ausführungsbeispiel wird bei jedem Motorzyklus eine Auswertung der Messung an der gesammelten Messmenge von Fluid vorgenommen oder aber es wird während einer vorgegeben Anzahl von Motorzyklen eine vorgegebene Messmenge an Fluid gesammelt, und eine Messung an der vorgegebenen Messmenge an Schmieröl durchgeführt und ausgewertet.

Bei Benutzung eines Messverfahrens der vorliegenden Erfindung kann beispielweise ein Wartungszeitpunkt für die Wartung einer vorgegebenen Motorkomponente, bevorzugt auch automatisch ermittelt werden, oder die Hubkolbenbrennkraftmaschine wird beispielweise in Abhängigkeit von einem Messergebnis an der gesammelten Messmenge von Schmieröl gesteuert und / oder geregelt.

Im Folgenden wird die Erfindung anhand weiterer Zeichnungen noch etwas eingehender erläutert. Es zeigen in schematischer Darstellung:
- Fig. 1:: eine Vorrichtung zur Optimierung des Schmierölfilms auf der Zylinderlauffläche;
- Fig. 2:: ein erstes einfaches Ausführungsbeispiel einer erfindungsgemässen Hubkolbenbrennkraftmaschine mit einer Überwachungsvorrichtung;
- Fig. 3:: ein zweites Ausführungsbeispiel gemäss Fig. 2 mit einer zusätzlichen Ölsammelöffnung im oberen Bereich des Zylinders;
- Fig. 4:: ein anderes Ausführungsbeispiel gemäss Fig. 2 mit mehreren Ölsammelöffnungen im Bereich der Spülschlitze;
- Fig. 5:: Eisengehalt einer Schmierölprobe in ppm;
- Fig. 6:: verbleibende Alkalinität einer Schmierölprobe in Milligramm KOH pro Gramm Schmierölprobe;
- Fig. 7:: ein erstes Beispiel eines Wassergehalts einer Schmierölprobe in Prozent der Probenmasse;
- Fig. 8:: ein zweites Beispiel eines Wassergehalts einer Schmierölprobe in Prozent der Probenmasse;

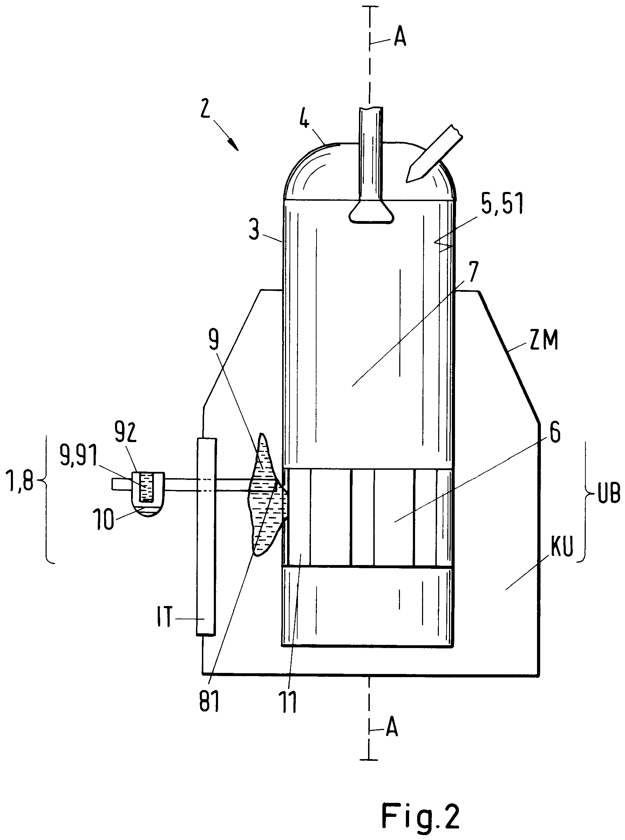

Da die Fig. 1 weiter oben bereits detailliert diskutiert wurde, kann im Folgenden direkt mit der Beschreibung der Fig. 2 fortgefahren werden.

Anhand der Fig. 2 ist ein erstes einfaches Ausführungsbeispiel einer erfindungsgemässen Hubkolbenbrennkraftmaschine mit einer Überwachungsvorrichtung schematisch dargestellt, die im folgenden gesamthaft mit dem Bezugszeichen 1 bezeichnet ist.

Die Überwachungsvorrichtung 1 der Fig. 2 zur Überwachung eines Verschleisszustands einer Komponente einer Hubkolbenbrennkraftmaschine 2 umfasst einen Zylinder 3 mit Zylinderdeckel 4 und einer an einer Zylinderwand 5 des Zylinders 3 vorgesehenen Lauffläche 51. Im Zylinder 3 ist ein Kolben 6 entlang der Lauffläche 51 zwischen einem unteren Totpunkt und einem oberen Totpunkt in einer axialen Richtung A derart hin- und herbewegbar angeordnet, dass der Kolben 6, der Zylinderdeckel 4 und die Zylinderwand 5 im Zylinder 3 einen Brennraum 7 zur Verbrennung eines Gemischs aus einem Treibstoff und Luft bilden. Dabei ist eine Ölsammeleinrichtung 8 zum Sammeln von Schmieröl 9 aus dem Zylinder 3 vorgesehen, so dass eine vorgebbare Messmenge 91 von Schmieröl 9 aus dem Zylinder 3 einer Messeinrichtung 10 zuführbar ist. Dabei ist in dem vorliegenden einfachen Ausführungsbeispiel der Fig. 2 exemplarisch nur eine Ölsammeleinrichtung 8 in einem unteren Bereich UB des Zylinders 3 im Bereich der Spülschlitze 11 vorgesehen. In Fig. 2 ist der Kolben 6 gerade im Bereich der Spülschlitze 11. Deutlich zu erkennen ist die Wolke aus Schmieröl 9, die aus dem Kolbenringpaket des Kolbens 6 durch die Spülschlitze 11 in den Raum des Zylindermantels ZM geblasen wird, welcher Raum des Zylindermantels ZM in an sich bekannter Weise durch die Inspektionstür IT erreichbar ist. Das Bezugszeichen KU bezeichnet den vertikalen Bereich der Kolbenunterseite. Hier wird die Messmenge 91, also eine Schmierölprobe 91 an Schmieröl 9 unmittelbar aus dem Kolbenringpaket des Kolbens 6 der Ölsammeleinrichtung 8 zugeführt und von einem Analyse- und Ansteuersystem, wie z.B. anhand der Fig. 1 erläutert, analysiert und mit Hilfe der Ergebnisse dieser Analysen dann die Schmierölzufuhr in den Zylinder 3 gesteuert und / oder geregelt.

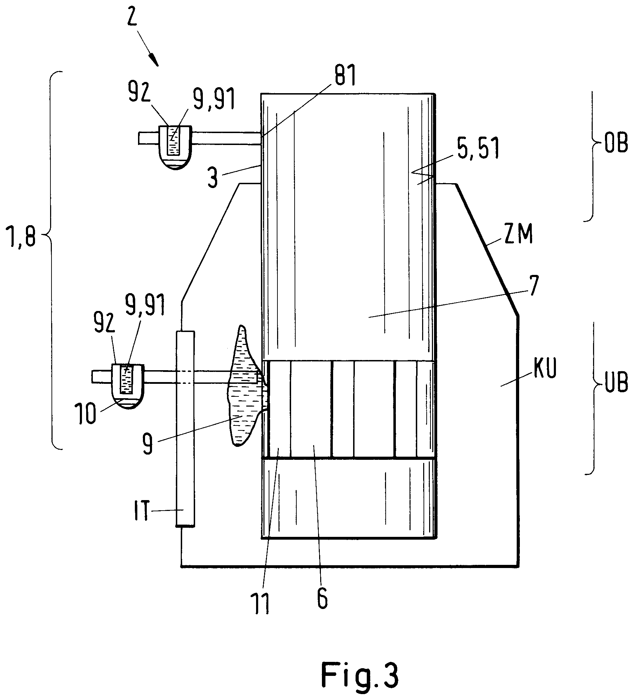

Die Fig. 3 zeigt ein zweites Ausführungsbeispiel gemäss Fig. 2 mit einer zusätzlichen Ölsammelöffnung 81 im oberen Bereich OB des Zylinders 3. Das Beispiel der Fig. 3 ist somit weitgehend identisch mit dem der Fig. 1. Mit Hilfe eines Ausführungsbeispiels gemäss Fig. 3 kann somit sowohl im unteren Bereich UB des Zylinders als auch im oberen Bereich OB des Zylinders Schmieröl 9 gesammelt werden.;

An dieser Stelle sei dabei ausdrücklich darauf hingewiesen, dass die Ölsammelöffnungen 81 im unteren Bereich UB nicht unbedingt im Bereich der Spülschlitze 11 angeordnet sein müssen, sondern in speziellen Fällen zum Beispiel auch oberhalb der Spülschlitze 11. Insbesondere ist es auch möglich, dass die erfindungsgemässe Überwachungsvorrichtung 1 an einem Motor ohne Spülschlitze vorgesehen ist. Zum Beispiel an einem Zweitakt- oder Viertakt-Motor mit einem Einlassventil.

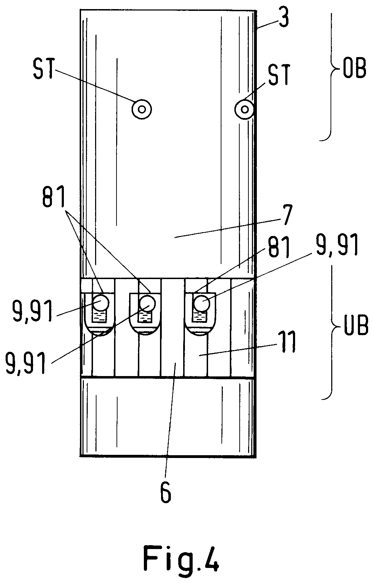

Anhand der Fig. 4 ist ein anderes Ausführungsbeispiel gemäss Fig. 2 mit mehreren Ölsammelöffnungen 81 im Bereich der Spülschlitze 11 schematisch dargestellt, wobei hier auch explizit im oberen Bereich OB des Zylinders 3 beispielhaft zwei Schmierölstellen ST dargestellt sind, auf deren Darstellung in den übrigen Figuren aus Gründen der Übersichtlichkeit verzichtet wurde. Wenn wie bei dem Ausführungsbeispiel der Fig. 4 in Umfangsrichtung verteilt mehrere Ölsammelöffnungen 81 vorgesehen sind, lässt sich vor allem in Umfangsrichtung eine homogene Verteilung der Schmierölqualität bzw. der Schmierölparameter mit der vorliegenden Erfindung realisieren, besonders gut auch dann, wenn in Umfangsrichtung verteilt am Zylinder 3 mehrere Schmierölstellen ST vorgesehen sind.

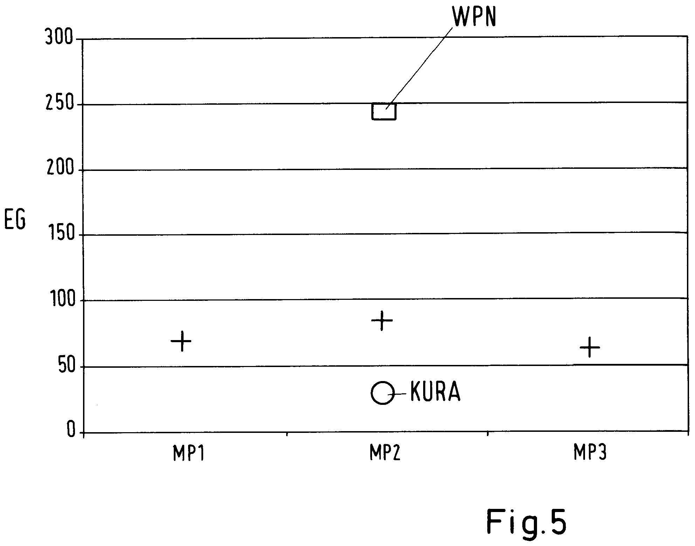

Die folgenden Fig. 5 bis Fig. 8 zeigen einige konkrete Messergebnisse, die mit Hilfe von erfindungsgemässen Überwachungsvorrichtungen in verschiedenen Laborversuchen erzielt wurden.

Fig. 5 zeigt den Eisengehalt EG einer Schmierölprobe in ppm (part per million), die an einer Ölsammelöffnung 81 von einer Zylinderwand WPN des Zylinders 3 genommen wurde. MP1 ist dabei eine Messposition 1, die am Spülschlitz 11, in Kolbenhubrichtung nicht unter einem Schmierstutzen vorgesehen war. MP2 ist eine Messposition 2 am Spülschlitz, die sich in Kolbenhubrichtung unter einem Schmierstutzen befindet. Und MP3 ist eine Messposition 3, die sich am Spülschlitz in Kolbenhubrichtung nicht unter einem Schmierstutzen befindet.

Die mit KURA bezeichneten Messpunkte entsprechen einem Kolben-Unterseiten-Raum-Abfluss.

Die Fig. 5 zeigt also, wie für einen bestimmten Motortyp, ausgerüstet mit einem bestimmten Schmiersystem und einem bestimmten System von Kolbenringen und einer dazu passenden Zylinderlinerbearbeitung, welches über eine bestimmte Zeit von einigen tausend Betriebsstunden eingelaufen ist, bei bestimmten Einstellungen des Schmiersystems sowie einer bestimmten Kombination von Motordrehzahl und Motorbelastung der Eisengehalt in der Schmierölprobe davon abhängt, wo am Motor die Probe entnommen wurde. Der Eisengehalt in der Schmierölprobe repräsentiert eine im Motorbetrieb auftretende Kombination aus abrasivem und korrosivem Verschleiss. Die Probe entnommen am Kolben-Unterseiten-Raum-Abfluss KUEA zeigt den tiefsten Eisengehalt, während alle Proben von den Messpositionen MP1 bis MP3, in Fig. 5 durch drei Kreuze im Diagramm eingetragen, am jeweiligen Spülschlitz einen signifikant höheren Wert zeigen und die Probe von der Entnahmeöffnung in der Zylinderwand den höchsten Wert zeigt. Die Messgenauigkeit beim Beispiel von Figur 5 liegt bei etwa 5 ppm. Dies wird so interpretiert, dass die Probe von der Entnahmeöffnung direkt in der Zylinderwand dem Zustand des Schmieröls auf der Zylinderwand am nächsten kommt, währenddem die Proben von den Messpositionen MP1 bis MP3 am jeweiligen Spülschlitz schon mit einer gewissen Menge von ungebrauchten Öl, welches mit jedem Kolbenhub durch die Kolbenringe von den Schmierstellen, welche sich im obersten Drittel des Zylinderliners befinden, nach unten zu den Spülschlitzen transportiert wird, vermischt sind und deswegen einen tieferen Eisengehalt zeigen, da frisches Schmieröl einen Eisengehalt von nur ca. 7 ppm aufweist. Die unterschiedlichen Eisengehalte an den Messpositionen MP1, MP2 und MP3 zeigen, dass das Schmieröl für den betrachteten Fall eine unterschiedliche Beschaffenheit aufweist, je nach dem wie sich die Messposition relativ zur Position der Schmierstellen im Zylinderliner verhält. Die Probe vom Kolben-Unterseiten-Raum-Abfluss weist den tiefsten Eisengehalt auf, was auf eine noch stärkere Vermischung mit frischem, ungebrauchtem Schmieröl hinweist, und darüber hinaus kann die Probe auch mit Schmieröl von benachbarten Zylindern verunreinigt sein.

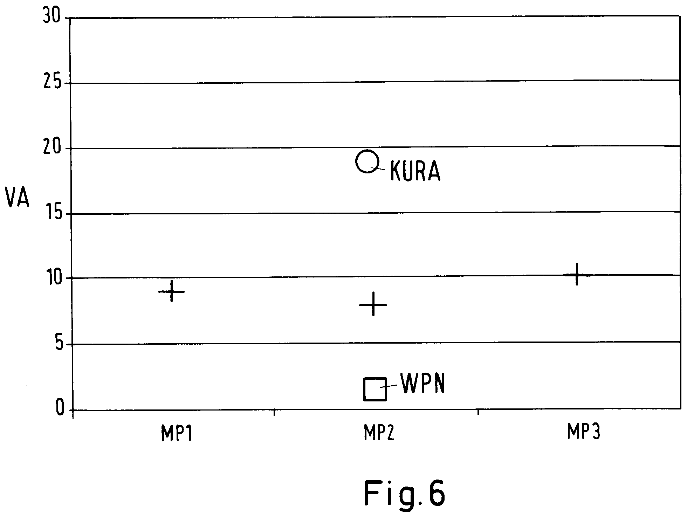

In Fig. 6 ist eine Messung der verbleibende Alkalinität VA einer Schmierölprobe in Milligramm KOH pro Gramm Schmierölprobe dargestellt Dabei ist VA die verbleibende Alkalinität (Basenzahl) der Ölprobe in Milligramm KOH pro Gramm Ölprobe, WPN ist die Wand-Probe-Nahme mittels einer Ölsammeleinrichtung in der Wand des Zylinders.

MP1 ist die Messposition 1 in einer Kolbenhub-Richtung, nicht unter einem Schmierstutzen befindlich, MP2 ist die Messposition 2 in einer Kolbenhub-Richtung unter einem Schmierstutzen befindlich und MP3 ist die Messposition 3 in Kolbenhub-Richtung nicht unter einem Schmierstutzen befindlich. MP1, MP2 und MP3 sind im Diagramm der Fig. 6 ebenso wie in Fig. 5 wieder durch Kreuze dargstellt. KURA ist auch hier wieder der Kolben-Unterseiten-Raum-Abfluss.

Fig. 6 zeigt, wie für einen bestimmten Motortyp, ausgerüstet mit einem bestimmten Schmiersystem und einem bestimmten System von Kolbenringen und einer dazu passenden Zylinderlinerbearbeitung, welches über eine bestimmte Zeit von einigen tausend Betriebsstunden eingelaufen ist, bei bestimmten Einstellungen des Schmiersystems sowie einer bestimmten Kombination von Motordrehzahl und Motorbelastung die verbleibende Alkalinität in der Schmierölprobe davon abhängt, wo am Motor die Probe entnommen wurde. Die dem frischen Schmieröl als Additiv beigefügte Alkalinität beträgt im Beispiel von Fig. 6 70 Milligramm KOH pro Gramm Schmieröl und dient der Neutralisation der im Motorbetrieb bei der Verbrennung von stark schwefelhaltigem Brennstoff entstehenden Schwefelsäure H₂SO₄. Die verbleibende Alkalinität in der Schmierölprobe repräsentiert die dem Öl noch innewohnende Fähigkeit, Schwefelsäure zu neutralisieren. Ist der Wert der verbleibenden Alkalinität in der Schmierölprobe auf oder gegen null abgesunken, kann keine oder keine genügende Neutralisation mehr stattfinden und das Material des Zylinderliners beginnt zu korrodieren, wodurch sich der Eisengehalt in der Schmierölprobe erhöht. Die Probe entnommen am Kolben-Unterseiten-Raum-Abfluss zeigt die höchste noch verbleibende Alkalinität, während alle Proben von den Messpositionen 1 bis 3 am jeweiligen Spülschlitz einen signifikant tieferen Wert zeigen und die Probe von der Entnahmeöffnung in der Zylinderwand einen Wert nahe bei null zeigt. Die Messgenauigkeit beim Beispiel von Figur 2 liegt bei etwa 1.5 Milligramm KOH pro Gramm Schmieröl. Dieses Resultat wird so interpretiert, dass die Probe von der Entnahmeöffnung direkt in der Zylinderwand dem Zustand des Schmieröls auf der Zylinderwand am nächsten kommt und somit die Gefahr von Korrosion der Zylinderoberfläche anzeigt, währenddessen die Proben von den Messpositionen 1 bis 3 am jeweiligen Spülschlitz schon mit einer gewissen Menge von ungebrauchten Öl, welches mit jedem Kolbenhub durch die Kolbenringe von den Schmierstellen, welche sich im obersten Drittel des Zylinderliners befinden, nach unten zu den Spülschlitzen transportiert wird, vermischt sind und deswegen einen höheren Gehalt an verbleibenden Alkalinität zeigen. Die unterschiedlichen Gehalte an verbleibender Alkalinität an den Messpositionen 1, 2 und 3 zeigen, dass das Schmieröl für den betrachteten Fall eine unterschiedliche Beschaffenheit aufweist, je nach dem wie sich die Messposition relativ zur Position der Schmierstellen im Zylinderliner verhält. Die Probe vom Kolben-Unterseiten-Raum-Abfluss weist den höchsten Gehalt an verbleibender Alkalinität auf, was auf eine noch stärkere Vermischung mit frischem, ungebrauchtem Schmieröl hinweist und darüber hinaus auch mit Schmieröl von benachbarten Zylindern verunreinigt sein kann.

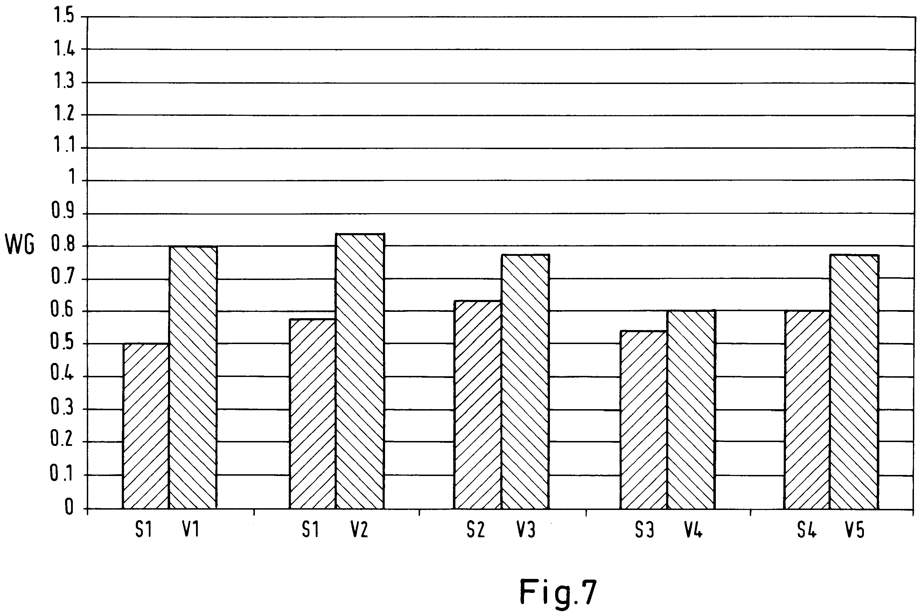

Die Fig. 7 zeigt ein erstes Beispiel eines Wassergehalts einer Schmierölprobe in Prozent der Probenmasse.

Dabei bedeuten die Bezeichnungen in Fig. 7:
WG = Wassergehalt der Ölprobe in Prozent der Probemasse
S1 = Schiff-Motor-Kombination 1
S2 = Schiff-Motor-Kombination 2
S3 = Schiff-Motor-Kombination 3
S4 = Schiff-Motor-Kombination 4
V1 = Zylinder-Schmierungs-Variante 1
V2 = Zylinder-Schmierungs-Variante 2
V3 = Zylinder-Schmierungs-Variante 3
V4 = Zylinder-Schmierungs-Variante 4
V5 = Zylinder-Schmierungs-Variante 5

Fig. 7 zeigt deutlich, dass der Wassergehalt in der Schmierölprobe, gebildet aus dem Mittelwert der Proben der in Figur 1 und 2 beschriebenen Messpositionen MP1 bis MP3, mit der Feuchtigkeit der für den Betrieb des Hubkolben-Verbrennungsmotors verwendeten Umgebungsluft korreliert, und zwar unabhängig vom für die Messung ausgewählten Schiff (insgesamt vier verschiedene) und unabhängig von der auf dem Motor auf dem ausgewählten Schiff installierten Zylinder-Schmierungs-Variante, bestehend aus einem bestimmten Schmiersystem, einem bestimmten System von Kolbenringen und einer dazu passenden Zylinderlinerbearbeitung sowie den Einstellungen des Schmiersystems. Ausser im Fall "S3 V4" war die Kombination von Motordrehzahl und Motorbelastung für alle in Fig. 7 gezeigten Fälle ähnlich, wodurch auf eine Lastabhängigkeit des Wassergehalts in der Schmierölprobe hingewiesen werden konnte.

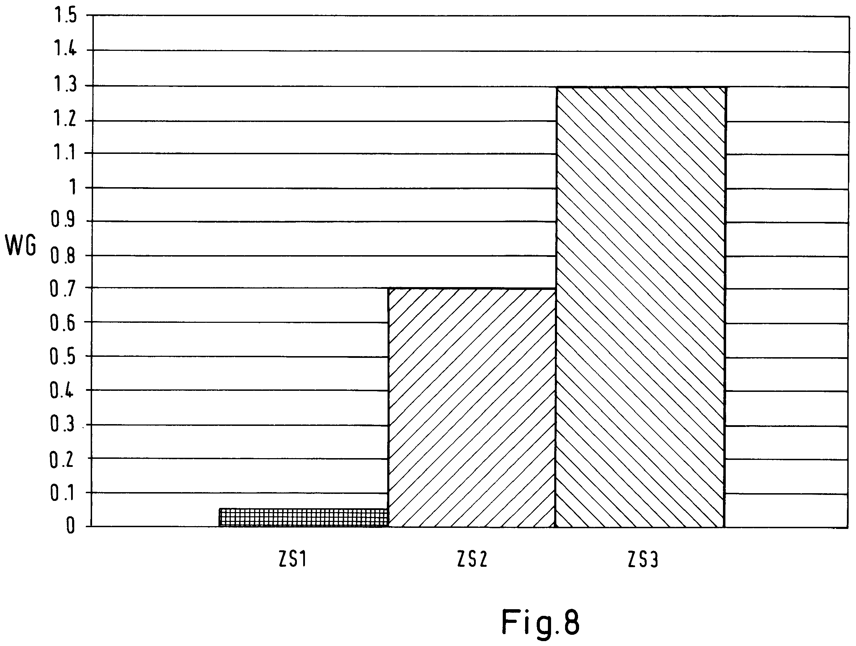

In Fig. 8 ist schliesslich eine Messung eines zweiten Beispiels eines Wassergehalts einer Schmierölprobe in Prozent der Probenmasse dargestellt, wobei WG wieder der Wassergehalt der Ölprobe in Prozent der Probemasse ist. ZS1 ist ungebrauchtes Zylinderschmieröl, ZS2 ist Zylinderschmieröl im stationären Betriebszustand und ZS3 Zylinderschmieröl im transienten Betriebszustand mit zunehmender Last.

Fig. 8 zeigt eindrucksvoll, dass der Wassergehalt in der Schmierölprobe, gebildet aus dem Mittelwert der Proben der in Figur 1 und 2 beschriebenen Messpositionen MP1 bis MP3, bei konstanter Feuchtigkeit der für den Betrieb des Hubkolben-Verbrennungsmotors verwendeten Umgebungsluft mit der Art der Motorbelastung korreliert, welche auf den Motor angewendet wird. Bei konstant gehaltenen Einstellungen des Schmiersystems wurde eine Messung ausgeführt (Zylinder-Schmieröl im stationären Betriebszustand), wo der Motor von 25% auf 50% der Nennlast gemäss Propellergesetz belastet wurde, dann die Last für 40 Minuten gehalten wurde, dann von 50% auf 75% der Nennlast gemäss Propellergesetz belastet wurde, dann die Last für 40 Minuten gehalten wurde, und schliesslich auf 100% der Nennlast belastet wurde, was insgesamt vier Stunden in Anspruch nahm. Bei einer anderen Messung mit dem selben Motor und den selben Einstellungen (Zylinder-Schmieröl im transienten Betriebszustand mit zunehmender Last) am selben Tag wurde der Motor von 25% kontinuierlich auf 100% der Nennlast belastet, was ungefähr 40 Minuten in Anspruch nahm. Es zeigt sich, dass die schnelle Belastung des Motors zu einem höheren Wassergehalt in der Schmierölprobe führt, was für das Verschleissverhalten des Zylinderliners als ungünstig beurteilt wird.

Es versteht sich von selbst, dass alle im Rahmen dieser Anmeldung beschriebenen Ausführungsbeispiel lediglich exemplarisch zu verstehen sind und insbesondere auch jede geeignete Kombination, bzw. jede dem Fachmann naheliegende Weiterbildung der Erfindung durch die Ansprüche abgedeckt sind.

## Patentansprüche

1. Hubkolbenbrennkraftmaschine mit einer Überwachungsvorrichtung zur Überwachung eines Verschleisszustands einer Komponente der Hubkolbenbrennkraftmaschine (2) umfassend einen Zylinder (3) mit Zylinderdeckel (4) und einer an einer Zylinderwand (5) des Zylinders (3) vorgesehenen Lauffläche (51), in welchem Zylinder (3) ein Kolben (6) entlang der Lauffläche (51) zwischen einem unteren Totpunkt und einem oberen Totpunkt in einer axialen Richtung (A) derart hin- und herbewegbar angeordnet ist, dass der Kolben (6), der Zylinderdeckel (4) und die Zylinderwand (5) im Zylinder (3) einen Brennraum (7) zur Verbrennung eines Gemischs aus einem Treibstoff und Luft bilden, wobei eine Ölsammeleinrichtung (8) zum Sammeln von Schmieröl (9) aus dem Zylinder (3) vorgesehen ist, so dass eine vorgebbare Messmenge (91) von Schmieröl (9) aus dem Zylinder (3) einer Messeinrichtung (10) zuführbar ist, und wobei die Ölsammeleinrichtung (8) eine an der Zylinderwand (5) zwischen dem unteren Totpunkt des Kolbens (6) und dem Zylinderdeckel (4) vorgesehene Ölsammelöffnung (81) umfasst, **dadurch gekennzeichnet, dass** die Messmenge (91) an Schmieröl (9) unmittelbar von der Lauffläche (51) des Zylinders (3) und unmittelbar aus einem Kolbenringpaket des Kolbens (6) der Ölsammeleinrichtung (8) zuführbar ist.

2. Hubkolbenbrennkraftmaschine nach Anspruch 1, wobei mindestens zwei Ölsammelöffnungen (81) vorgesehen sind, die in Umfangsrichtung und / oder in Bezug auf die axiale Richtung (A) zueinander versetzt am Zylinder (3) angeordnet sind.

3. Hubkolbenbrennkraftmaschine nach Anspruch 1 oder 2, wobei die Ölsammeleinrichtung (8) ein von einer Steuereinrichtung betätigbares Ölsammelventil umfasst.

4. Hubkolbenbrennkraftmaschine nach einem der vorangehenden Ansprüche, wobei die Hubkolbenbrennkraftmaschine (2) eine Viertakt-Hubkolbenbrennkraftmaschine (2) ist und die Ölsammelöffnung (81) derart am Zylinder (3) zwischen der Position einer Krone des Kolbens (6) im oberen Totpunkt und der Position einer Unterseite des Kolbens (6) im unteren Totpunkt angeordnet ist, so dass Schmieröl (9) aus dem Zylinder (3) durch die Ölsammelöffnung (81) sammelbar ist.

5. Hubkolbenbrennkraftmaschine nach einem der vorangehenden Ansprüche, wobei die Hubkolbenbrennkraftmaschine (2) eine längs gespülte Brennkraftmaschine (2) mit Spülschlitzen (11), insbesondere ein langsam laufender Zweitakt-Grossdieselmotor (2) ist, und die Ölsammelöffnung (81) derart im Bereich der Spülschlitze (11) angeordnet ist, dass über die Spülschlitze (11) austretendes Schmieröl (9) durch die Ölsammelöffnung (81) sammelbar ist.

6. Hubkolbenbrennkraftmaschine nach einem der vorangehenden Ansprüche, wobei die Ölsammeleinrichtung (8) einen Ölsammelraum (92) umfasst, der bevorzugt ein integraler Bestandteil der Zylinderwand (5) ist, und im Ölsammelraum (92) gesammeltes Schmieröl (9) der Messeinrichtung (10) zuführbar ist.

7. Hubkolbenbrennkraftmaschine nach einem der vorangehenden Ansprüche, wobei die Ölsammelöffnung (81) derart ausgestaltet ist, dass ein Schmieröldurchsatz einstellbar ist.

8. Hubkolbenbrennkraftmaschine nach einem der vorangehenden Ansprüche, wobei Messeinrichtung (10) eine elektromagnetische Messeinheit umfasst, insbesondere eine Messeinheit zur amplitudenabhängigen und / oder frequenzabhängigen und / oder frequenzunabhängigen Bestimmung einer Kapazität, einer magnetischen Permeabilität, einer elektrischen Gleichstromleitfähigkeit und / oder Wechselstromleitfähigkeit, und / oder einer komplexen elektrischen Leitfähigkeit und / oder eines komplexen elektrischen Widerstands der gesammelten Messmenge (91) von Schmieröl (9) umfasst, wobei die Überwachungsvorrichtung bevorzugt miniaturisiert direkt in der Zylinderwand (5) vorgesehen sein kann.

9. Hubkolbenbrennkraftmaschine nach einem der vorangehenden Ansprüche, wobei die Messeinrichtung (10) eine Röntgenmesseinheit umfasst, insbesondere zur Bestimmung einer Transmissionseigenschaft und / oder einer Absorptionseigenschaft und / oder einer Reflexionseigenschaft und / oder einer Fluoreszenzeigenschaft der gesammelten Messmenge (91) von Schmieröl (9).

10. Hubkolbenbrennkraftmaschine nach einem der vorangehenden Ansprüche, wobei die Messeinrichtung (10) eine optische Messeinheit zur Bestimmung einer optischen Transmissionseigenschaft und / oder einer optischen Absorptionseigenschaft und / oder einer optischen Reflexionseigenschaft und / oder einer optischen Fluoreszenzeigenschaft Eigenschaft der gesammelten Messmenge (91) von Schmieröl (9) umfasst, wobei die optische Messeinheit bevorzugt eine Infrarot Messeinheit und / oder eine Ultraviolett Messeinheit ist.

11. Hubkolbenbrennkraftmaschine nach einem der vorangehenden Ansprüche, wobei die Messeinrichtung (10) eine chemische Messeinheit zur Bestimmung einer chemischen Zusammensetzung der gesammelten Messmenge (91) von Schmieröl (9) umfasst.

12. Hubkolbenbrennkraftmaschine nach einem der vorangehenden Ansprüche, wobei die Messeinrichtung (10) ein Messsystem zur Bestimmung eines Gehaltes an Wasser und / oder eines Gehaltes an Metall, insbesondere Eisen und / oder Chrom und / oder Vanadium, und / oder zur Bestimmung eines Gehaltes an Phosphor und / oder Schwefel der gesammelten Messmenge (91) von Schmieröl (9) umfasst.

13. Hubkolbenbrennkraftmaschine nach einem der vorangehenden Ansprüche, ausgestaltet als Zweitakt-Grossdieselmotor oder Viertakt-Motor.

14. Überwachungsverfahren zur Überwachung eines Verschleisszustands einer Komponente einer Hubkolbenbrennkraftmaschine (2) umfassend einen Zylinder (3) mit Zylinderdeckel (4) und einer an einer Zylinderwand (5) des Zylinders (3) vorgesehenen Lauffläche (51), in welchem Zylinder (3) ein Kolben (6) entlang der Lauffläche (51) zwischen einem unteren Totpunkt und einem oberen Totpunkt in einer axialen Richtung (A) derart hin- und herbewegbar angeordnet ist, dass der Kolben (6), der Zylinderdeckel (4) und die Zylinderwand (5) im Zylinder (3) einen Brennraum (7) zur Verbrennung eines Gemischs aus einem Treibstoff und Luft bilden, wobei eine Ölsammeleinrichtung (8) zum Sammeln von Schmieröl (9) aus dem Zylinder (3) vorgesehen wird, und im Betriebszustand eine vorgebbare Messmenge (91) von Schmieröl (9) aus dem Zylinder (3) einer Messeinrichtung (10) zugeführt wird, und wobei die Ölsammeleinrichtung (8) eine an der Zylinderwand (5) zwischen dem unteren Totpunkt des Kolbens (6) und dem Zylinderdeckel (4) vorgesehene Ölsammelöffnung (81) umfasst, **dadurch gekennzeichnet, dass** die Messmenge (91) an Schmieröl (9) unmittelbar von der Lauffläche (51) des Zylinders (3) und unmittelbar aus einem Kolbenringpaket des Kolbens (3) der Ölsammeleinrichtung (8) zugeführt wird.

15. Überwachungsverfahren nach Anspruch 14, wobei die Ölsammelöffnung (81) derart ausgestaltet wird, dass ein Schmieröldurchsatz eingestellt, bevorzugt automatisch eingestellt werden kann.

## Claims

1. A reciprocating piston internal combustion engine having a monitoring apparatus for monitoring a state of wear of a component of the reciprocating piston internal combustion engine (2) including a cylinder (3) having a cylinder cover (4) and a running surface (51) provided at a cylinder wall (5) of the cylinder (3), in which cylinder (3) a piston (6) is arranged moveable to and fro in an axial direction (A) along the running surface (51) between a bottom dead center and a top dead center such that the piston (6), the cylinder cover (4) and the cylinder wall (5) form a combustion space (7) in the cylinder (3) for the combustion of a mixture of a fuel and air, wherein an oil collection device (8) is provided for the collection of lubrication oil (9) from the cylinder (3) so that a predetermined measured quantity (91) of lubrication oil (9) is suppliable from the cylinder (3) to a measurement device (10), and wherein the oil collection device (8) includes an oil collection opening (81) which is provided at the cylinder wall (5) between the bottom dead center of the piston (6) and the cylinder cover (4), **characterized in that** the measured quantity (91) of lubrication oil (9) is directly suppliable from the running surface (51) of the cylinder (3) and is directly suppliable from a piston ring package of the piston (6) to the oil collection device (8).

2. A reciprocating piston internal combustion engine in accordance with claim 1, wherein at least two oil collection openings (81) are provided which are arranged at the cylinder (3) displaced from one another in the circumferential direction and/or with regard to the axial direction (A).

3. A reciprocating piston internal combustion engine in accordance with claim 1 or 2, wherein the oil collection device (8) includes an oil collection valve actuatable by a control device.

4. A reciprocating piston internal combustion engine in accordance with any one of the preceding claims, wherein the reciprocating piston internal combustion engine (2) is a four-stroke reciprocating piston internal combustion engine (2) and the oil collection opening (81) is arranged at the cylinder (3) between the position of a crown of the piston (6) in the top dead center and the position of a bottom side of the piston (6) in the bottom dead center such that lubrication oil (9) is collectable from the cylinder (3) through the oil collection opening (81).

5. A reciprocating piston internal combustion engine in accordance with any one of the preceding claims, wherein the reciprocating piston internal combustion engine (2) is a longitudinally scavenged internal combustion engine (2) having scavenging slits (11), in particular a slow running two-stroke large diesel engine (2) and the oil collection opening (81) is arranged in the region of the scavenging slits (11) such that lubrication oil (9) exiting via the scavenging slits (11) is collectable through the oil collection opening (81).

6. A reciprocating piston internal combustion engine in accordance with any one of the preceding claims, wherein the oil collection device (8) includes an oil collection space (92) which is preferably an integral part of the cylinder wall (5) and lubrication oil collected in the oil collection space (92) is suppliable to the measurement device (10).

7. A reciprocating piston internal combustion engine in accordance with any one of the preceding claims, wherein the oil collection opening (81) is designed such that a lubrication oil flow-rate is settable.

8. A reciprocating piston internal combustion engine in accordance with any one of the preceding claims, wherein the measurement device (10) includes an electromagnetic measurement unit, in particular includes a measurement unit for the amplitude dependent and/or the frequency dependent and/or the frequency independent determination of a capacitance, of a magnetic permeability, of an electric DC conductivity and/or of an AC conductivity, and/or of a complex electric conductivity and/or of a complex electric resistance of the collected measured quantity (91) of lubrication oil (9), wherein the monitoring apparatus is preferably provided miniaturized directly in the cylinder wall (5).

9. A reciprocating piston internal combustion engine in accordance with any one of the preceding claims, wherein the measurement device (10) includes an X-ray measurement unit, in particular for the determination of a transmission property and/or of an absorption property and/or of a reflection property and/or of a fluorescence property of the collected measured quantity (91) of lubrication oil (9).

10. A reciprocating piston internal combustion engine in accordance with any one of the preceding claims, wherein the measurement device (10) includes an optical measurement unit for the determination of an optical transmission property and/or of an optical absorption property and/or of an optical reflection property and/or of an optical fluorescence property of the collected measured quantity (91) of lubrication oil (9), wherein the optical measurement unit is preferably an infrared measurement unit and/or an ultraviolet measurement unit.

11. A reciprocating piston internal combustion engine in accordance with any one of the preceding claims, wherein the measurement device (10) includes a chemical measurement unit for the determination of a chemical composition of the collected measured quantity (91) of lubrication oil (9).

12. A reciprocating piston internal combustion engine in accordance with any one of the preceding claims, wherein the measurement device (10) includes a measurement system for the determination of a content of water and/or of a content of metal, in particular of iron and/or of chromium and/or of vanadium, and/or for the determination of a content of phosphor and/or of sulfur of the collected measured quantity (91) of lubrication oil (9).

13. A reciprocating piston internal combustion engine in accordance with any one of the preceding claims, designed as a two-stroke large diesel engine or as a four-stroke engine.

14. A monitoring method for monitoring a state of wear of a component of a reciprocating piston internal combustion engine (2) including a cylinder (3) having a cylinder cover (4) and a running surface (51) provided at a cylinder wall (5) of the cylinder (3), in which cylinder (3) a piston (6) is arranged moveable to and fro in an axial direction (A) along the running surface (51) between a bottom dead center and a top dead center such that the piston (6), the cylinder cover (4) and the cylinder wall (5) form a combustion space (7) in the cylinder (3) for the combustion of a mixture of a fuel and air, wherein an oil collection device (8) is provided for the collection of lubrication oil (9) from the cylinder (3) and in the operating state a predetermined measured quantity (91) of lubrication oil (9) is supplied from the cylinder (3) to a measurement device (10), and wherein the oil collection device (8) includes an oil collection opening (81) which is provided at the cylinder wall (5) between the bottom dead center of the piston (6) and the cylinder cover (4), **characterized in that** the measured quantity (91) of lubrication oil (9) is directly supplied from the running surface (51) of the cylinder (3) and is directly supplied from a piston ring package of the piston (3) to the oil collection device (8).

15. A monitoring method in accordance with claim 14, wherein the oil collection opening (81) is designed such that a lubrication oil flow-rate can be set, preferably such that a lubrication oil flow-rate can be set automatically.

## Revendications

1. Un moteur à combustion interne à piston alternatif avec un dispositif de surveillance pour la surveillance d'un état d'usure d'un composant du moteur à combustion interne à piston alternatif (2), comprenant un cylindre (3) avec un couvercle de cylindre (4) et une surface de roulement (51) prévue sur une paroi de cylindre (5) du cylindre (3), dans lequel cylindre (3) un piston (6) est disposé le long de la surface de roulement (51) de manière à pouvoir être déplacé en va-et-vient dans une direction axiale (A) entre un point mort inférieur et un point mort supérieur, de telle manière que le piston (6), le couvercle de cylindre (4) et la paroi de cylindre (5) forment une chambre de combustion (7) dans le cylindre (3) pour la combustion d'un mélange d'un carburant et d'air, dans lequel un dispositif collecteur d'huile (8) est prévu pour collecter l'huile lubrifiante (9) du cylindre (3) de telle sorte qu'une quantité mesurée (91) prédéterminable d'huile lubrifiante (9) peut être alimentée par le cylindre (3) à un dispositif de mesure (10), et dans lequel le dispositif collecteur d'huile (8) comprend une ouverture collecteur d'huile (81) prévue sur la paroi du cylindre (5) entre le point mort inférieur du piston (6) et le couvercle du cylindre (4), **caractérisé en ce que** la quantité mesurée (91) d'huile lubrifiante (9) peut être alimentée directement de la surface de roulement (51) du cylindre (3) et directement d'un paquet de segments de piston du piston (6) au dispositif collecteur d'huile (8).

2. Un moteur à combustion interne à piston alternatif selon la revendication 1, dans lequel au moins deux dispositifs collecteurs d'huile (8) sont prévus, qui sont disposés sur le cylindre (3) décalés l'un par rapport à l'autre dans la direction circonférentielle et / ou par rapport à la direction axiale (A).

3. Un moteur à combustion interne à piston alternatif selon la revendication 1 ou 2, dans lequel le dispositif collecteur d'huile (8) comprend une vanne de collection d'huile, qui peut être actionnée par un dispositif de commande.

4. Un moteur à combustion interne à piston alternatif selon l'une des revendications précédentes, dans lequel le moteur à combustion interne à piston alternatif (2) est un moteur à combustion interne à piston alternatif (2) à quatre temps et l'ouverture collecteur d'huile (81) est disposée sur le cylindre (3) entre la position d'une couronne du piston (6) au point mort supérieur et la position d'une face inférieure du piston (6) au point mort inférieur de sorte que d'huile lubrifiante (9) peut être collecté du cylindre (3) par l'ouverture collecteur d'huile (81).

5. Un moteur à combustion interne à piston alternatif selon l'une des revendications précédentes, dans lequel le moteur à combustion interne à piston alternatif (2) est un moteur à combustion interne (2) purgé longitudinalement avec des fentes de purge (11), en particulier un grand moteur diesel (2) à deux temps fonctionnant lentement, et l'ouverture collecteur d'huile (81) est disposée dans la zone des fentes de purge (11) de telle sorte que d'huile lubrifiante (9) sortant par les fentes de purge (11) peut être collectée par l'ouverture de collecteur d'huile (81).

6. Un moteur à combustion interne à piston alternatif selon l'une des revendications précédentes, dans lequel le dispositif collecteur d'huile (8) comprend une chambre collecteur d'huile (92), qui est de préférence une partie intégrante de la paroi du cylindre (5), et d'huile lubrifiante (9) collectée dans la chambre collecteur d'huile (92) peut être alimentée au dispositif de mesure (10).

7. Un moteur à combustion interne à piston alternatif selon l'une des revendications précédentes, dans lequel l'ouverture de collecteur d'huile (81) est configurée de telle sorte qu'un débit d'huile lubrifiante peut être réglé.

8. Un moteur à combustion interne à piston alternatif selon l'une des revendications précédentes, le dispositif de mesure (10) comprenant une unité de mesure électromagnétique, en particulier comprenant une unité de mesure pour la détermination d'une capacité dépendant de l'amplitude et / ou dépendant de la fréquence et / ou indépendante de la fréquence, d'une perméabilité magnétique, d'une conductivité électrique d'un courant continu et / ou d'une conductivité d'un courant alternatif, et / ou d'une conductivité électrique complexe et / ou d'une résistance électrique complexe de la quantité mesurée (91) collectée d'huile lubrifiante (9), dans lequel le dispositif de surveillance peut être prévu de préférence sous forme miniaturisée directement dans la paroi du cylindre (5).

9. Un moteur à combustion interne à piston alternatif selon l'une des revendications précédentes, le dispositif de mesure (10) comprenant une unité de mesure à rayons X, en particulier pour la détermination d'une propriété de transmission et / ou d'une propriété d'absorption et / ou d'une propriété de réflexion et / ou d'une propriété de fluorescence de la quantité mesurée (91) collectée d'huile lubrifiante (9).

10. Un moteur à combustion interne à piston alternatif selon l'une des revendications précédentes, le dispositif de mesure (10) comprenant une unité de mesure optique pour la détermination d'une propriété de transmission optique et /ou d'une propriété d'absorption optique et / ou d'une propriété de réflexion optique et / ou d'une propriété de fluorescence optique de la quantité mesurée (91) collectée d'huile lubrifiante (9), dans lequel l'unité de mesure optique est de préférence une unité de mesure infrarouge et / ou une unité de mesure ultraviolette.

11. Un moteur à combustion interne à piston alternatif selon l'une des revendications précédentes, le dispositif de mesure (10) comprenant une unité de mesure chimique pour la détermination d'une composition chimique de la quantité mesurée (91) collectée d'huile lubrifiante (9).

12. Un moteur à combustion interne à piston alternatif selon l'une des revendications précédentes, le dispositif de mesure (10) comprenant un système de mesure pour la détermination d'une teneur en eau et / ou d'une teneur en métal, en particulier en fer et / ou chrome et / ou vanadium, et / ou pour la détermination d'une teneur en phosphore et / ou soufre de la quantité mesurée (91) collectée d'huile lubrifiante (9).

13. Un moteur à combustion interne à piston alternatif selon l'une des revendications précédentes, configuré comme un grand moteur diesel à deux temps ou comme un moteur à quatre temps.

14. Une procédure de surveillance pour la surveillance d'un état d'usure d'un composant d'un moteur à combustion interne à piston alternatif, comprenant un cylindre (3) avec un couvercle de cylindre (4) et une surface de roulement (51) prévue sur une paroi de cylindre (5) du cylindre (3), dans lequel cylindre (3) un piston (6) est disposé le long de la surface de roulement (51) de manière à pouvoir être déplacé en va-et-vient dans une direction axiale (A) entre un point mort inférieur et un point mort supérieur, de telle manière que le piston (6), le couvercle de cylindre (4) et la paroi de cylindre (5) forment une chambre de combustion (7) dans le cylindre (3) pour la combustion d'un mélange d'un carburant et d'air, dans lequel un dispositif collecteur d'huile (8) est prévu pour collecter d'huile lubrifiante (9) du cylindre (3), et à l'état de fonctionnement, une quantité mesurée (91) prédéterminable d'huile lubrifiante (9) est alimentée par le cylindre (3) à un dispositif de mesure (10), et dans lequel le dispositif collecteur d'huile (8) comprend une ouverture collecteur d'huile (81) prévue sur la paroi du cylindre (5) entre le point mort inférieur du piston (6) et le couvercle du cylindre (4), **caractérisé en ce que** la quantité mesurée (91) d'huile lubrifiante (9) est alimentée directement de la surface de roulement (51) du cylindre (3) et directement d'un paquet de segments de piston du piston (6) au dispositif collecteur d'huile (8).

15. Une procédure de surveillance selon la revendication 14, dans lequel l'ouverture collecteur d'huile (81) est configurée de telle sorte qu'un débit d'huile lubrifiante peut être réglé, de préférence réglé automatiquement.
